# EUROPEAN PATENT SPECIFICATION

(11) **EP 3 427 949 B1**
(45) Date of publication and mention of the grant of the patent: **09.10.2024**
(21) Application number: 17181083.1
(22) Date of filing: 12.07.2017
(51) Int. Cl.: B33Y 70/00, A61L 27/20, C12N 5/00, C08L 5/12

(54) **MECHANICALLY TUNABLE BIOINKS FOR BIOPRINTING**
MECHANISCH ABSTIMMBARE BIOTINTEN ZUM BIODRUCKEN
BIO-ENCRES MÉCANIQUEMENT SYNTONISABLES POUR BIO-IMPRESSION

(43) Date of publication of application: 16.01.2019
(73) Proprietor: MAPTECH Holdings UG, 79206 Breisach am Rhein (DE)
(72) Inventor: SHASTRI, Prasad V., 79206 Breisach (DE); FORGET, Aurelien, 4059 Queensland (AU); BLAESER, Andreas, 53840 Troisdorf (DE); FISCHER, Horst, 52074 Aachen (DE)
(74) Representative: Krauss, Jan

(56) References cited:
- EP-A1- 2 735 318
- WO-A1-2012/055596
- WO-A1-2014/072035
- WO-A1-2016/092106
- WO-A1-2016/100856
- US-A1- 2016 122 723

## Description

The present invention relates to the use of mechanically tunable bioinks for bioprinting and respective processes.

In recent years, it has become evident that the biophysical aspects (mechanics, topography) of the extracellular matrix (ECM) can impact cell phenotype and function. Since organs are composed of different cell types the mechanical properties show spatial variations as each cell type evolves in its own specialized ECM and the elastic modulus of biological tissue therefore exhibits huge diversity and ranges in general from 100 to 100 000 Pa.

Materials with mechanical properties that can be adjusted to reproduce the natural ECM therefore are highly valuable.

In order to generate 3D organized, cell-laden ECM scaffolds, different biofabrication techniques have evolved in the past decade. Broadly, these can be categorized into two approaches: (1) generation of a 3D scaffold followed by association with cell population (i.e. cell seeding) and (2) direct fabrication of 3D constructs comprising cells dispersed in a gelable medium - the so called bioink.

In the latter approach, which is commonly referred to as bioprinting, biological material is three-dimensionally structured, usually by means of a gel. Commonly used processes for bioprinting are so called inkjet-printing, so called syringe-printing or bioplotting and so-called laser-printing.

Bioprinting poses high demands on the cytocompatibility of the material, offers, however, the advantage to generate constructs with spatially defined cell and material composition.

Moreover, in some cases biomaterials used in cell culturing (such as alginate, gelatin, nanocellulose or agarose) offer some advantages for bioprinting as they are soluble in water and can be formulated as a cell carrier.

In bioprinting the bioink is dispensed through a printer head and as the bioink is dispensed, it is exposed to shear forces and the fluid-induced shear stress resulting therefrom can be detrimental to the cells. For this reason, the printing process requires optimization of dispensing parameters.

Several natural polymers have been explored for bioprinting, including alginate, gelatin and nanocellulose. Agarose also has been described recently as bioink for the 3D printing of umbilical artery endothelial cells. However, tailoring the mechanical properties of agarose requires changing the concentration of the agarose which modification has a profound impact on the rheological properties of agarose. The same applies to other polysaccharides.

As a consequence, the printing parameters have to be adjusted to the viscosity in order to yield reproducible results and droplets of consistent size and, secondly, changes in the viscosity and the printing parameters alter the fluid-induced shear stress experienced by cells.

WO 2012/055596 and EP 2735318 describe extracellular matrices comprising modified polysaccharides. The matrices are characterized by comprising modified primary hydroxyl groups containing polysaccharides comprising repeating disaccharide units wherein in at least part of the disaccharide units the primary hydroxyl group is replaced by functional groups selected from carboxyl groups, halide groups or groups comprising sulfur or phosphorus atoms, like e.g. sulfate groups, sulfonate groups, phosphonate groups and phosphate groups.

WO 2014/072035 A1 discloses sulfated alginates different from the present invention for tissue engineering, wherein a cell may be embedded.

WO 2016/092106 A1 discloses syringe bioprinting with a bioink comprising cells, such as cartilage cells or induced pluripotent stem cells, and biopolymers, but not agarose.

WO 2016/100856 A1 discloses a bioink comprising stem cells and cellulose.

US 2016/122723 discloses 3D printing with bioinks, agarose is listed with the possible bioink biopolymers.

It was an object of the present invention to provide materials for 3D bioprinting that overcome or at least reduce the problems associated with the use of known materials in bioprinting.

This object is achieved by the use in accordance with claim 1 and the process in accordance with claim 14 of the appended claims.

Preferred embodiments of the present invention are described in the dependent claims and in the detailed specification hereinafter.

In accordance with the present invention, a matrix comprising a modified primary hydroxyl groups containing polysaccharide comprising repeating disaccharide units wherein in at least part of the disaccharide units the primary hydroxyl group is replaced by functional groups selected from carboxyl groups, halide groups or groups comprising sulfur or phosphorus atoms, like e.g. sulfate groups, sulfonate groups, phosphonate groups and phosphate groups, for bioprinting processes, characterized in that the modified primary hydroxyl groups containing polysaccharide is agarose is used for bioprinting.

As used herein, the term "polysaccharide" relates to a polymeric carbohydrate structure, which is formed of repeating units joined together by glycosidic bonds. Preferably, the repeating units are either mono- or disaccharides and the polymeric structure of the polysaccharide is non-branched. It is preferred that the number average molecular weight of the polysaccharide ranges from 10 000 Dalton to 500 000 Dalton, particularly preferred the number average molecular weight of the polysaccharide ranges from 50 000 Dalton to 300 000 Dalton, with a number average molecular weight of the polysaccharide ranging from 80 000 Dalton to 140 000 Dalton being even more preferred.

Agar, the main source of agarose, is a structural polysaccharide of the cell walls of a variety of red seaweed. Important sources of agar are *Gelidiaceae* such as *Gelidium amansii, Gelidium japonicum, Gelidium pacificum, Gelidium subcostatum, Pterocladia tenuis* and *Acanthopeltis japonica,* red algae belonging to *Gracilariaceae* such as *Gracilaria verrucosa* and *Gracilaria gigas,* red algae belonging to *Ceramiaceae* such as *Ceramium kondoiand Campylaephora hypnaeoides.* Agar consists of two groups of polysaccharides, namely agarose and agaropectin. Agarose is a neutral, linear polysaccharide with no branching and has a backbone consisting of 1,3-linked β-D-galactose-(1-4)-a-L-3,6 anhydrogalactose repeating units. This dimeric repeating unit, called agarobiose differs from a similar dimeric repeating unit called carrabiose which is derived from carrageenan in that it contains 3,6-anhydrogalactose in the L-form and does not contain sulfate groups.

The dimeric repeating units derived from the polysaccharides are chemically modified by the specific modification of the primary hydroxyl group into a functional group selected from carboxyl groups, halide groups or groups comprising sulfur or phosphorus atoms, like e.g. sulfate groups, sulfonate groups, phosphonate groups and phosphate groups. Suitable processes for such modification are described in WO 2012/055596 and EP 2735318.

In accordance with a preferred embodiment, the functional group of the modification is a carboxyl group, i.e. carboxylated polysaccharides are preferably used.

Depending on the reaction conditions it is possible to modify a certain percentage of the primary hydroxyl groups. According to the invention, preferably at least 5 %,more preferably at least 11% of the disaccharide units are modified. In an even more preferred embodiment at least 20 up to 99% of the disaccharide repeat units of the primary alcohol groups are modified into a functional group as defined above. In a particularly preferred embodiment in 50-95% of the repeat disaccharide units the primary alcohol group is modified into a functional group as defined above.

In order to determine as precisely as possible the percentage of the modified primary alcohol groups it is possible either to perform the modification reaction in a controlled manner or alternatively the polysaccharide is modified completely so that about 100% of the primary alcohol groups are modified. Such completely modified polysaccharide can be blended with unmodified polysaccharide which may either be the same polysaccharide or another polysaccharide. Since the chemical modification can be precisely controlled and by controlling the blending with another polysaccharide or the same unmodified polysaccharide it is possible to control the chemical properties of the extracellular matrix.

One important aspect is the shear modulus which can range from about 10 Pa which reflects the structure of a nerve tissue to about 10⁷ Pa which corresponds with the shear modulus of cartilage tissues. By blending gels of different extent of chemical modification the nanoscale structure of the gel can be impacted. It has been shown that nanoscale topography influences cell shape, cytoskeletal assembly and function. The shear modulus of the extracellular matrix ranges preferably from 1 Pa to 100 kPa and more preferred from 1 Pa to 50 kPa and in a most preferred embodiment in a range from 10 Pa to 10 kPa.

In an especially preferred embodiment agarose, wherein the primary alcohol group has been oxidized in a carboxylic acid group, is blended with non-modified agarose.

To obtain a halide modification, the unmodified polysaccharide may be reacted with N-halogenated succinimide in dry dimethylformamide using triphenyl phosphine as catalyst. The following reaction scheme shows this general reaction for agarose as polysaccharide and Br as preferred halide:

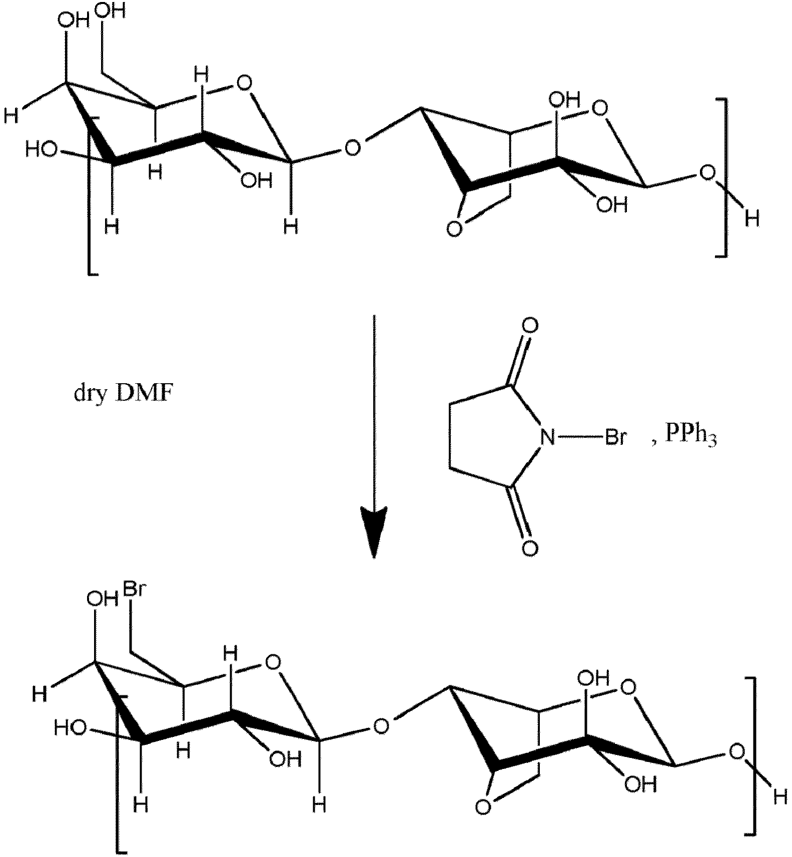

A versatile reagent for the nucleophilic fluorination of primary alcohols is diethyl amino sulfur trifluoride (DAST), which is commercially available e.g. from Sigma Aldrich. DAST has proven to be versatile in a significant number of fluorinations of in particular alcohol groups and thus it can be used to obtain the fluorinated polysaccharides in accordance with the present invention. The skilled person will choose the suitable reaction conditions based on his professional knowledge.

Ritter et. al in Current Topics in Drug Discovery and Development 2008, 11 (6) 803ff. provide a good review of fluorination reactions with various reagents including DAST.

The introduction of halides into the backbone of the polysaccharide promotes intramolecular interaction within the polysaccharide chain.

To obtain a modification with phosphate groups, the polysaccharides may be reacted with phosphoric acid in dry DMSO in the presence of urea in accordance with the following reaction scheme (again shown for agarose):

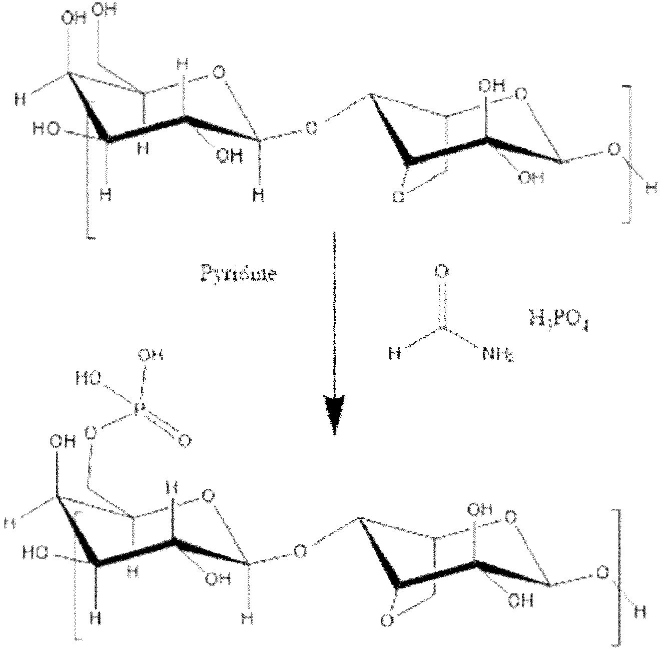

The introduction of sulfate groups may be achieved by reacting the respective polysaccharide with a combination of chlorosulfonic acid and formamide in dry pyridine in accordance with the following reaction scheme (again shown for agarose):

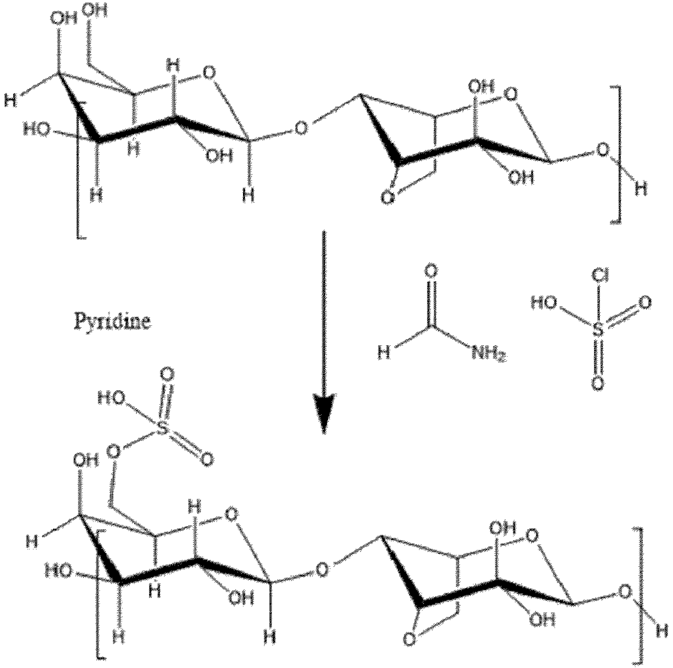

The introduction of charged groups like the sulfate or phosphate groups into the backbone of the polysaccharide impacts the intermolecular chain-chain hydrogen bonding interaction in the polysaccharide chain due to an increase in the electronic repulsion.

Sulfonation appears to eliminate inter-molecular hydrogen bonding to a significant extent.

Depending on the chosen reaction conditions it is possible to adjust the degree of modification of the primary hydroxyl groups in the polysaccharide. In accordance with a preferred embodiment at least approximately 5, more preferably at least 10 % of the primary hydroxyl groups are modified with the respective halogen or phosphorus or sulfur containing groups. Even more preferably 20 to 99 % of the primary hydroxyl groups are modified with a range of 50 to 95 % of modification being even more preferred.

Thus, the modification reaction may be carried out in a controlled manner so that only a partial modification of the primary hydroxyl groups of the primary alcohol groups takes place. However, the polysaccharide can also be modified in such a way that about 100% of the primary hydroxyl groups are modified.

The completely or partially modified polysaccharide can be subsequently blended with an unmodified polysaccharide, which may either be the same polysaccharide or another polysaccharide. Since the nature and extent of the chemical modification of the modified polysaccharide can be controlled and the blending ratio with another polysaccharide or the same unmodified polysaccharide can be adjusted it is possible to control the chemical properties of the resulting matrix.

The presence of charged moieties like sulfonate or phosphate groups along the backbone of the polysaccharide chain has been observed to interrupt or weaken the chain-chain interaction leading to a decrease of the shear modulus compared to the respective modulus of the unmodified polysaccharide whereas the introduction of halide groups may be used to increase the modulus G'.

It has been found that extracellular matrices comprising the modified polysaccharides as described before, are particularly suitable for use in bioprinting, i.e. they can preferably be used as components of so called bioinks.

The term bio-ink as used herein denotes a material comprising living cells that behaves much like a liquid, allowing people to "print" it in order to create a desired shape. To make bio-ink, usually a slurry of cells that can be loaded into a cartridge and inserted into a specially designed printer, along with another cartridge containing a gel is formulated.

The term bioprinting, when used herein, denotes a process wherein biological material is three-dimensionally structured, usually by means of a gel. In bioprinting, there are three major types of printers that have been used. These are inkjet, laser-assisted, and extrusion printers. Inkjet printers are mainly used in bioprinting for fast and large-scale products. One type of inkjet printer, called drop-on-demand inkjet printer, prints materials in exact amounts, minimizing cost and waste. Printers that utilize lasers provide high-resolution printing; however, these printers are often expensive. Extrusion printers print cells layer-by-layer, just like 3D printing to create 3D constructs. In addition to just cells, extrusion printers may also use hydrogels infused with cells.

3D bioprinting is the process of creating cell patterns in a confined space using 3D printing technologies, where cell function and viability are preserved within the printed construct. Usually, 3D bioprinting utilizes the layer-by-layer method to deposit materials known as bioinks to create tissue-like structures that are later used in medical and tissue engineering fields.

3D bioprinting for fabricating biological constructs typically involves dispensing cells onto a biocompatible scaffold using a successive layer-by-layer approach to generate tissue-like three-dimensional structures. Artificial organs such as livers and kidneys made by 3D bioprinting have been shown to lack crucial elements that affect the body such as working blood vessels, tubules for collecting urine, and the growth of billions of cells required for these organs. Without these components the body has no way to get the essential nutrients and oxygen deep within their interiors. Given that every tissue in the body is naturally compartmentalized of different cell types, many technologies for printing these cells vary in their ability to ensure stability and viability of the cells during the manufacturing process. Some other methods than inkjet, syringe or laser printing that are used for 3D bioprinting of cells are photolithography, magnetic bioprinting, stereolithography, and direct cell extrusion.

Commonly used processes for bioprinting are so called inkjet-printing, so called syringe-printing or bioplotting and so-called laser-printing.

In the inkjet printing method, cells in a liquid are propelled onto a carrier by means of a piezo nozzle. This process works similarly to the commercial paper-inkjet printing process, with the sole exception that a biological ink in the form of a carrier liquid with cells suspended therein is used instead of ink for printing. By means of this process, extremely fine amounts can be printed. Since the drops produced by the piezo-nozzles are propelled with biological cells in the carrier liquid from the piezo-nozzles and must fly through the air over the distance between the ends of the piezo-nozzles and the surfaces intended to receive the object to be printed. The drops undergo deformations in flight, causing them to undergo a wobbling motion during the flight. This can lead to certain inaccuracies with such an inkjet printer, as the cells do not always reach their intended location. Furthermore, a three-dimensional layered structure of the object to be produced is somewhat limited, as layering usually can only be carried out from above, making it difficult to produce support structures and overhanging structures.

Such inkjet printing methods are described for example in WO 99/48541 , US 2009/0208466 , US 2011/0076734, and US 2011/0250688 .

In the syringe printing method, the material to be printed is loaded into a syringe and forced out of the syringe by means of compressed air or punching pressure. The nozzle of the syringe in this case is moved into its intended position by an x-y-z movement unit in accordance with the object to be printed. A specified amount of the printing material is then pressed out of the syringe at the site intended for printing. In this manner, a three-dimensional object composed of layers is produced. The advantage of this method is a simple structure, but precise dosing usually is a somewhat complex process. Moreover, if different cells are to be used to make up the structure of the three-dimensional object, further syringes should be kept in reserve and used for printing in addition to or instead of the first syringe. This increases both the structural complexity of a corresponding printer and the time required for the actual printing. Ultimately, this is reflected in an increase in costs.

Syringe printing methods of this kind are described for example in WO 2013/113883, US 2011/0313542, US 8,580,546, and US 2012/0288938 .

US 2014/0093932 also describes a syringe printing method in which additional curing of the biological material already accumulated at the intended site is carried out by means of UV light.

In the laser printing method, printing is carried out by means of a laser beam. Here, a carrier film is initially coated with a liquid containing cells. After this, a laser beam pulse is directed onto the coated carrier film, causing a drop of the cell suspension to be propelled from the carrier film. The individual drops can then be stacked atop one another by means of skilled stacking - similar to the inkjet printing method. It is true that the amount of the drops of the cell suspension to be applied can be dosed with quite high accuracy. However, the drops deform during the flying phase on their way to the surface on which the object to be printed is to be produced. Because of the wobbling movement connected therewith, this in turn can result in inaccuracies in positioning the drops. As the drop size as a whole is rather small, the laser printing method is also comparatively slow.

As may be understood form the foregoing, there is a variety of bioprinting processes known to the skilled person and he will select the best suitable process type based on his professional experience and taking into account the specific application case. Respective processes and machinery have also been described in the literature so that no further details need to be given here.

In the course of the present invention, it has been surprisingly found that extracellular matrices comprising modified polysaccharides as described hereinbefore are particularly useful for use in bioprinting, i.e. as components of bioinks.

The compressive strength of gels comprising modified polysaccharides as defined above, can be tailored to span the range of natural tissues while exhibiting the same viscosity under printing conditions by varying the degree of modification. As a result, 3D structures comprised of stiff and soft hydrogels can be produced under identical printing conditions. This unique property of the modified polysaccharides described hereinbefore ensures significantly reduced shear stress induced cell death compared to the printing of equally concentrated native polysaccharides of the same type.

It appears that the gelation of the modified polysaccharides which presumably occurs through physical association of helices allows to decouple the viscosity of the respective bioink solution from its mechanical properties.

Some other benefits are now described for agarose.

The gelling behavior of agarose is characterized by a hysteresis, that is, the transition from solution to gel (T_{sol-gel}) occurs at lower temperature than the gel-to-solution transition (T_{gel-sol}). Upon cooling below T_{sol-gel} the gelation process is triggered as double-stranded helices aggregate through hydrogen bonding (H-bonding). Agarose gels of different compressive strength can be realized by controlling the density of helices by varying the polymer concentration. In contrast, the hysteresis and thermal transitions in native polysaccharide hydrogels show no dependence on concentration.

In order to print, the bioink has to be a liquid - and in the solution state the viscosity of native polysaccharides is strongly dependent on concentration. The viscosity of unmodified polysaccharide gels, different to gels comprising modified polysaccharides as used in the present invention, cannot be decoupled from their mechanical properties in the gel state.

In the modified agarose, the polymer chains are heterogenous and possess two secondary structures (helical and sheet-like). The gelation of the modified polysaccharides is dictated by the helical content and the degree of modification influences this content (increasing degree of modification reduces the helical content). Increasing the degree of modification decreases both the solution-to-gel and the gel-to-solution temperature. However, unexpectedly, the viscosity of the polysaccharides with different degree of modification of the primary hydroxyl groups is only modestly and much less influenced compared to native polysaccharides.

It appears that the content having a sheet-like secondary structure does not participate in gellation and this at the end affords bioinks based on such modified polysaccharides with different mechanical properties in gel phase but similar solution viscosities and even more independently of the polysaccharide concentration, which is a significant and unexpected advantage for the use in bioprinting.

A further advantage of the modified polysaccharides compared to native unmodified polysaccharides when used as bioinks is the better consistency of droplet volumes.

For bioprinting experiments in this regard, native agarose and carboxylated agarose bioinks were loaded into a heated container that maintained the hydrogel above T_{sol-gel}. A computer-controlled microvalve (300 µm nozzle diameter) at a pressure of 50 kPa was used for droplet generation. Opening the microvalve for different gating times allowed expulsion of drops of different sizes. Following expulsion, the droplet rapidly cooled below the solution to gel temperature and formed a hydrogel. By successive addition of droplet layers, complex 3D structures were assembled. In order to assess the quality and precision of the droplet the average droplet volume of native agarose and carboxylated agarose formulations for different gating times (450, 600, and 900 ms) were compared. While the droplet volume generally increased with increasing gating time to a certain degree, this effect was much more pronounced for native agarose formulations compared to carboxylated agarose formulations. This is an important attribute, as consistent droplet volumes across the different formulations facilitates the printing of 3D matrices comprising of the multiple formulations.

In order to build 3D structures with defined mechanical domains, bioinks upon gelation need to retain their positional accuracy. The precision of the placement of the bioink was tested by building a complex 3D structure composed of a helical channel of carboxylated agarose with 60 % modification (CA60) embedded in a modified agarose cylinder with 28 % modification (CA28). In order to distinguish between the two hydrogel structures, CA60 was labeled with the fluorescent dye Rhodamine 123. The ability to print such channels of soft hydrogel within a stiffer hydrogel could be used to present different mechanical cues to a single population of cells to invoke different differentiation programs based on the cell microenvironment.

Since fluid flow induced shear stress that can damage cell membrane and compromise cell viability, the nozzle shear stress for native agarose and the carboxylated agarose formulations was determined using a fluid-dynamics mode. Resolution of the fluid-dynamics model for a 300 µm nozzle shows that native agarose has the highest calculated shear stress (6.1 kPa), whereas in the carboxylated agarose formulations, this was substantially lower (1.6-2.5 kPa). To ascertain the suitability of the CA bioinks and microvalve printer in printing cells, human mesenchymal stem cells (hMSCs) isolated from femoral heads of three independent donors (n = 3) were pooled and dispersed in 2% w/v solutions of native agarose, CA28, CA60, and CA93 at 37° C (10⁶ cells ml⁻¹). The hMSC viability was assessed immediately after printing using a live/dead assay and compared to hMSCs simply physically dispersed within the hydrogel. hMSCs were chosen as they have been shown to respond to mechanical cues. The total number of dead cells per field of view (FOV) was calculated and plotted alongside the shear stress, and this showed increased cell death with increasing shear stress. Comparison of cell viability in printed versus nonprinted native agarose hydrogel revealed that cell viability was significantly lower in the printed native agarose gels (65%). However, cells embedded in CA28 and CA60 could be printed with negligible change in their viability (95 and 91%, respectively) in comparison to cells in nonprinted controls. Surprisingly, the viability of cells printed in CA93 (carboxylated agarose with 93 % carboxyl modification) was statistically lower than the control. This small drop in viability may be attributed to higher shear stress experienced by cells in the CA93 during dispensation. Nevertheless, the viability of cells in the printed CA93 was still substantially higher (81%) in comparison to those printed in native agarose (65%).

In order to ascertain the proliferation status of hMSCs after printing, hMSCs were dispersed in native agarose and in CA60, and the cell numbers immediately after printing were counted in a fixed field of view (FOV 2.8 m x 2.2 mm) and compared to cell numbers after 7 d. Since native agarose and carboxylated agarose both lack cell adhesion domains, hMSCs were also printed using CA60 supplemented with 0.16%w/v Collagen 1 (Col-1). Po]ysaccharide hydrogels such as carboxylated agarose that show limited cell adherence have been modified with cell adhesion peptides or blended with silk fibroin to promote cell adhesion, and likewise NA has been blended with collagen to formulate an extrudable bioink that can support cell proliferation. Carboxylated agarose was blended with collagen to improve bioink cell interactions. While a statistically significant increase in the number of live cells, assessed using live/dead staining, was observed in native agarose, the increase observed in the case of CA60 was not significant. However, CA60 supplemented with Col-1 showed a tenfold higher cell numbers in comparison to day 0, that is, immediately after printing. Since the addition of Col-1 does not modify the mechanical properties of carboxylated agarose, it appears that the printing conditions do not impact hMSC proliferation and thus printed CA bioink supports the postprinting viability of cells for at least 7 d.

Since the nozzle viscosity of the carboxylated agarose bioink is decoupled from its mechanical properties in the gel state, the suitability of CA28 and CA60 to render complex hydrogel structures was investigated. hMSCs dispersed in CA60 were printed as three straight and three curved channels in different planes within a cylinder of CA28. Fluorescent microscopy images revealed that the two hydrogels were indeed spatially resolved with cells dearly confined in one of the channels. The successful printing of such structures demonstrates the ability to fabricate complex 3D structures of different mechanical and biological properties using bioink comprising modified polysaccharides.

Flow-induced shear stress is an inevitable physical effect present in every dispensing process, for example, pipetting, extrusion, and droplet ejection. The level of shear stress is a vital criterion for postprinting cell survival and proliferation in 3D bioprinting. The nozzle geometry and the rheological properties of the applied hydrogel were identified as the most powerful driving forces that determine the level of shear stress in a dispensing system. In the present invention, it has been shown that chemical modification of hydrogels, such as carboxylation, is an effective method to tune the rheological properties of a bioink in order to reduce dispensation associated shear stress-induced cell death. Further to changing the material properties, the printing process can be adjusted to minimize shear stress. One such example is spraying of cell-laden droplets using an electric field - electrohydrodynamic spraying This technique has been further developed for cell encapsulation within an electrospun fiber that can be organized into a precise scaffold, and it has been shown that these cells remain viable upon transplantation in mice. Nevertheless, electrohydrodynamic spraying, bioextrusion, and bioplotting require the cells be suspended or dispersed in a material that can be ejected through a nozzle. This will invariably induce a shear stress, which can be detrimental to the cell viability. The more viscous the solution is, the higher will be the shear stress. Therefore, having a bioink with a low viscosity is beneficial for techniques that require an extrusion of cell suspension within a biomaterial. In this context, the bioink used in accordance with the present invention where the bioink solution rheology is decoupled from bioink gel phase properties is potentially useful in the above-mentioned processes and can be advantageously applied where shear stress associated cell death is an issue.

In the course of the present invention it has been demonstrated that thermoresponsive and mechanically tailorable modified polysaccharides, in particular carboxylated agarose, can be utilized as bioinks to print complex architectures in which cells can be precisely organized within hydrogels of different stiffness. Such structured 3D environments could allow the spatial guidance of cell differentiation by patterned mechanical cues within a hydrogel environment and enable the incorporation of mechanobiology paradigms in the direct 3D bioprinting of cells.

### Examples

Native agarose was obtained from Merck (Darmstadt, Germany), (2,2,6,6-tetramethylpiperidin-1-yl)oxyl (TEMPO, 99%), sodium bromide (NaBr, 99%), NaOCI solutions 15% v/v, NaBH₄ (99.99%), NaCl (BioXtra > 99.5%), Rhodamine 123, and phosphate buffer saline (PBS) were purchased from Sigma Aldrich (USA) and used as received. Ethanol technical grade was used as received. Deionized water was obtained from a laboratory ion exchanger.

*Synthesis of Carboxylated Agarose:* 1 g of native agarose type 1 (Merck) was transferred into a three-necked round bottom flask, equipped with a mechanical stirrer and pH meter. The reactor was heated to 90°C to dissolve the agarose and then cooled to 0°C in an ice bath under mechanical stirring to prevent the solution from gelling. The reactor was then charged with TEMPO (0.160 mmol, 20.6 mg), NaBr (0.9 mmol, 0.1 g), and NaOCI (2.5 mL, 15% v/v solution) under vigorous stirring. The pH of the solution was adjusted to pH 10.8 throughout the duration of the reaction, and the degree of carboxylation was controlled by the addition of predetermined volumes of NaOH solution (0.5 M). At the end of the reaction NaBH₄ (0.1 g) was added, and the solution was acidified to pH 8 and stirred for 1 h. The carboxylated agarose was precipitated by sequential addition of NaCl (0.2 mol, 12 g) and ethanol (500 mL), and the solid was collected by vacuum filtration and extracted using ethanol. Residual ethanol was removed by extensive dialysis against water and the carboxylated agarose was obtained as a white solid upon freeze-drying overnight.

*Rheological Characterization: The* viscosities of native agarose and carboxylated agarose solutions/hydrogels were characterized using a rotary rheometer (Kinexus, Malvern Instruments, Worcestershire, UK) with a 4 cone and plate geometry. Shear stress and viscosity were measured for shear rates from 0.01 to 10 000 s¹ at 40°C. The viscosity was measured for different temperatures, from 60 to 5°C with cooling at the rate of 1° C min⁻¹, with a constant shear rate of 10 000 s⁻¹.

*Mechanical Testing:* Native agarose (NA) and carboxylated agarose (CA) bulk hydrogels were cast in a 15 mm diameter cylindrical plastic vial and allowed to set overnight at 4°C. The bottom of the vial was cut open and the cylinder of hydrogel was pushed out of the vial. Hydrogel discs were cut to similar height and measured using a caliper before testing. Printed hydrogel samples were produced with an in-house developed 3D printer. A 15 mm diameter cylinder was printed with a height of 10 mm. The hydrogels were allowed to set overnight at 4°C prior to testing. Compression testing of printed and bulk samples was carried out on a universal testing machine (Zwick, Germany) equipped with a 50 N load cell at a speed of 1 cm min⁻¹ until fracture of the hydrogels, and the data were exported and analyzed using Microsoft Excel (Microsoft, Redmond, WA).

*Droplet Volume:* The volume of the droplet dispensed by the printer for different NA and CA formulations (NA 0.5, 1 and 2% w/v; 2% w/v CA28, CA60, and CA93) was determined by extruding 500 droplets into a tared 1.5 mL Eppendorf tube. The total weight of each dispensation (500 droplets) through a 300 nozzle for various gating times (450, 675, and 900 ms) was measured for three independent dispensations.

3D *Printer* The printer comprised four printer heads mounted to a three-axis robotic system (Isel, Eichenzell, Germany). Each head could be heated, pressurized, and controlled individually (Figure S6A, Supporting Information). The printer heads were composed of an electromagnetic microvalve (Fritz Gyger, Gwatt, Switzerland) connected to a pressurized bioink reservoir. The printing pressure could be varied from 0 to 300 kPa. For all experiments the air pressure was adjusted to 50 kPa. The printer head was designed to enable quick exchange of the attached microvalves between valves with small (150 µm), medium (300 µm), and big (600 µm) nozzle diameters. The microvalve constitutes the basic dispensing unit of the system. By application of an electric current running through a magnetic coil, the valve ball is lifted magnetically against the mechanical force of a spring. The valve opens and allows a fraction of hydrogel-cell suspension to be squeezed out of the nozzle. The gating time could be varied from 450 µs to 1 sec. By dropping the current, the magnetic force was reduced and the ball was pressed into the seat again closing the valve. The printing stage was cooled by circulating refrigerant cooled down to -10 °C using a cooling aggregate (TC45-F, Peter Huber Kältemaschinenbau, Offenburg, Germany). The print head carrying the carboxylated agarose formulation was heated up to 40°C and the printing pressure set to 50 kPa. All 3D bioprinting experiments with Rhodamine stained hydrogels and with loaded cells were conducted using the 300 µm nozzle. After printing the structures were held for 20 min at 4°C to ensure the complete gelation of agarose.

*Postprinting Viability Study on hMSCs:* Four different hydrogel precursor solutions were prepared by mixing 0.04 g mL⁻¹ NA or CA (28%, 60%, and 93%) with phosphate buffered saline (PBS). The agarose solutions were subsequently autoclaved at 121°C for 15 min. Four million hMSCs from three independent donors (n = 3) were pooled together and resuspended in 2 mL growth medium (Mesenpan; PAN Biotech, Aidenbach, Germany) supplemented with 2%v/v fetal calf serum (FCS) and 1 % v/v solution of 10000 units of penicillin mixed with 10 mg streptomycin (Gibco, Life Technologies, Carlsbad, CA). The hMSCs were isolated from femoral heads of three independent donors. For determining cell viability, 500 µL of each of the NA or CA solution was mixed at 37°C and resuspended with equal volumes of the cell suspension resulting in a final cell concentration of 106 cells/mL.

The hM SC dispersed in either NA or CA formulation was loaded into the printer head and printed dropwise into a 96-well plate. For each type of hydrogel, three samples with a final volume of 100 uL were printed using the 300 um microvalve (Fritz Gyger, Gwatt, Switzerland) at an air pressure of 50 kPa and a valve gating time of 450 ms. As a control three samples with a volume of 100 µL of each hydrogel type were pipetted into the well plate. Immediately after printing cell viability was assessed using a vital fluorescence staining assay. The staining solution contained 0.083 mg/mL 1-propidium iodide (P4170-10116, Sigma-Aldrich, St. Louis, MO) and 0.083 mg/rnL-1 fluorescein diacetate (F7378-10G, Sigma-Aldrich, St. Louis, MO) in Ringer's solution. Fach sample was imaged three times using an inverted microscope (DM16000B, Leica Microsystems. Wetzlar, Germany). Living and dead cells were counted using imaged.

*Shear Stress during Printing.* The shear stress the cells were exposed to during the dispensing process was estimated using fluid dynamics model. In addition to the printing settings, nozzle size (300 µm) and air pressure (50 kPa), the flow consistency index (K) and the flow behavior index (η), which describe the rheological behavior of a hydrogel solution, were applied as input parameters for shear stress calculations. Values of K and η were derived from the viscosity measurements of the hydrogel solutions.

## Claims

1. Use of a matrix comprising a modified primary hydroxyl groups containing polysaccharide comprising repeating disaccharide units wherein in at least part of the disaccharide units the primary hydroxyl group is replaced by functional groups selected from carboxyl groups, halide groups or groups comprising sulfur or phosphorus atoms, like e.g. sulfate groups, sulfonate groups, phosphonate groups and phosphate groups, for bioprinting processes, **characterized in that** the modified primary hydroxyl groups containing polysaccharide is agarose.

2. Use in accordance with claim 1 wherein the bioprinting process is selected from inkjet-printing, syringe-printing or bioplotting and laser-printing.

3. Use in accordance with claim 1 or 2 wherein the functional group is a carboxyl group.

4. Use in accordance with claim 1 or 2 wherein the functional group is a halide group.

5. Use in accordance with claim 1 or 2 wherein the functional group is a phosphonate or phosphate group.

6. Use in accordance with claim 1 or 2 wherein the functional group is a sulfate or sulfonate group.

7. Use in accordance with any of the preceding claims wherein the matrix additionally comprises unmodified polysaccharides.

8. Use in accordance with any of the preceding claims wherein either the modified polysaccharide or the non-modified polysaccharide or both are selected from the group consisting of a member of the carrageenan family, hyaluronic acid, and agarose.

9. Use in accordance with claim 8 wherein both the modified polysaccharide and the non-modified polysaccharide are agarose.

10. Use in accordance with any of the preceding claims wherein in the modified polysaccharides at least 5 % of the primary hydroxyl groups are converted into a functional group.

11. Use of a bioink formulation comprising a modified primary hydroxyl groups containing polysaccharide comprising repeating disaccharide units wherein in at least part of the disaccharide units the primary hydroxyl group is replaced by functional groups selected from carboxyl groups, halide groups or groups comprising sulfur or phosphorus atoms, like e.g. sulfate groups, sulfonate groups, phosphonate groups and phosphate groups, **characterized in that** the modified primary hydroxyl groups containing polysaccharide is agarose, and living cells, for bioprinting or bioprinting processes.

12. The use in accordance with claim 11, wherein the living cells are selected from the group consisting of chondrocytes, osteoblasts, osteoclasts, skin epithelial cells, intestinal epithelial cells, corneal epithelial cells, astrocytes, neurons, oligodendrocytes, smooth muscle cells, endothelial cells, cardiomyocytes, pancreatic islet cells, kidney epithelial cells and naive cells obtained from umbilical cord.

13. The use in accordance with claim 11 or 12, wherein the living cells are stem cells selected from the group consisting of embryonic stem cells, somatic stem cells, reprogrammed pluripotent somatic cells, induced pluripotent cells and amniotic stem cells.

14. A process for three-dimensionally structuring biological materials, wherein a bioink formulation comprising a modified primary hydroxyl groups containing polysaccharide comprising repeating disaccharide units wherein in at least part of the disaccharide units the primary hydroxyl group is replaced by functional groups selected from carboxyl groups, halide groups or groups comprising sulfur or phosphorus atoms, like e.g. sulfate groups, sulfonate groups, phosphonate groups and phosphate groups, **characterized in that** the modified primary hydroxyl groups containing polysaccharide is agarose, and living cells, is processed by extrusion printing, inkjet-printing, syringe-printing or bioplotting or laser-printing, wherein the inkjet-printing is preferably drop-on-demand inkjet printing.

15. The process according to claim 14, wherein the living cells are selected from the group consisting of chondrocytes, osteoblasts, osteoclasts, skin epithelial cells, intestinal epithelial cells, corneal epithelial cells, astrocytes, neurons, oligodendrocytes, smooth muscle cells, endothelial cells, cardiomyocytes, pancreatic islet cells, kidney epithelial cells and naive cells obtained from umbilical cord, or wherein the living cells are stem cells selected from the group consisting of embryonic stem cells, somatic stem cells, reprogrammed pluripotent somatic cells, induced pluripotent cells and amniotic stem cells.

## Patentansprüche

1. Verwendung einer Matrix umfassend ein modifizierte primäre Hydroxylgruppen enthaltendes Polysaccharid umfassend sich wiederholende Disaccharideinheiten, worin in mindestens einem Teil der Disaccharideinheiten die primäre Hydroxylgruppe durch funktionelle Gruppen ausgewählt aus Carboxylgruppen, Halidgruppen oder Gruppen umfassend Schwefel- oder Phosphoratome, wie zum Beispiel Sulfatgruppen, Sulfonatgruppen, Phosphonatgruppen und Phosphatgruppen ersetzt ist, für Biodruckverfahren, **dadurch gekennzeichnet, dass** das modifizierte primäre Hydroxylgruppen enthaltende Polysaccharid Agarose ist.

2. Verwendung in Übereinstimmung mit Anspruch 1, wobei das Biodruckverfahren ausgewählt ist aus Inkjetdruck, Spritzendruck oder Bioplotting und Laserdrucken.

3. Verwendung in Übereinstimmung mit Anspruch 1 oder 2, wobei die funktionelle Gruppe eine Carboxylgruppe ist.

4. Verwendung in Übereinstimmung mit Anspruch 1 oder 2, wobei die funktionelle Gruppe eine Halidgruppe ist.

5. Verwendung in Übereinstimmung mit Anspruch 1 oder 2, wobei die funktionelle Gruppe eine Phosphonat- oder Phosphatgruppe ist.

6. Verwendung in Übereinstimmung mit Anspruch 1 oder 2, wobei die funktionelle Gruppe eine Sulfat- oder Sulfonatgruppe ist.

7. Verwendung in Übereinstimmung mit einem der voranstehenden Ansprüche, wobei die Matrix zusätzlich nicht-modifizierte Polysaccharide umfasst.

8. Verwendung in Übereinstimmung mit einem der voranstehenden Ansprüche, wobei entweder das modifizierte Polysaccharid oder das nicht-modifizierte Polysaccharid oder beide ausgewählt sind aus der Gruppe bestehend aus einem Mitglied der Carrageen Familie, Hyaluronsäure und Agarose.

9. Verwendung in Übereinstimmung mit Anspruch 8 wobei sowohl das modifizierte Polysaccharid und das nicht-modifizierte Polysaccharid Agarose sind.

10. Verwendung in Übereinstimmung mit einem der voranstehenden Ansprüche, wobei in den modifizierten Polysacchariden mindestens 5 % der primären Hydroxylgruppen in eine funktionelle Gruppe umgewandelt sind.

11. Verwendung einer Biotintenformulierung umfassend ein modifizierte primäre Hydroxylgruppen enthaltendes Polysaccharid umfassend sich wiederholende Disaccharideinheiten, worin in mindestens einem Teil der Disaccharideinheiten die primäre Hydroxylgruppe durch funktionelle Gruppen ausgewählt aus Carboxylgruppen, Halidgruppen oder Gruppen umfassend Schwefel- oder Phosphoratome, wie zum Beispiel Sulfatgruppen, Sulfonatgruppen, Phosphonatgruppen und Phosphatgruppen ersetzt ist, **dadurch gekennzeichnet, dass** das modifizierte primäre Hydroxylgruppen enthaltende Polysaccharid Agarose ist, und lebende Zellen, zum Biodrucken oder in Biodruckverfahren.

12. Verwendung in Übereinstimmung mit Anspruch 11, wobei die lebenden Zellen ausgewählt sind aus der Gruppe bestehend aus Chondrocyten, Osteoblasten, Osteoclasten, Hautepithelzellen, intestinalen Epithelzellen, Cornea-Epithelzellen, Astrocyten, Neuronen, Oligodendrocyten, glatten Muskelzellen, Endothelzellen, Cardiomyocyten, Pankreas-Inselzellen, Nieren-Epithelzellen und naiven Zellen erhalten aus der Nabelschnur.

13. Verwendung in Übereinstimmung mit Anspruch 11 oder 12, wobei die lebenden Zellen Stammzellen sind, ausgewählt aus der Gruppe bestehend aus embryonalen Stammzellen, somatischen Stammzellen, reprogrammierten pluripotenten somatischen Zellen, induzierten pluripotenten Zellen und amniotischen Stammzellen.

14. Verfahren zum dreidimensionalen Strukturieren von biologischen Materialien, wobei eine Biotinte-Formulierung umfassend ein modifizierte primäre Hydroxylgruppen enthaltendes Polysaccharid umfassend sich wiederholende Disaccharideinheiten, worin in mindestens einem Teil der Disaccharideinheiten die primäre Hydroxylgruppe durch funktionelle Gruppen ausgewählt aus Carboxylgruppen, Halidgruppen oder Gruppen umfassend Schwefel- oder Phosphoratome, wie zum Beispiel Sulfatgruppen, Sulfonatgruppen, Phosphonatgruppen und Phosphatgruppen ersetzt ist, **dadurch gekennzeichnet, dass** das modifizierte primäre Hydroxylgruppen enthaltende Polysaccharid Agarose ist, und lebende Zellen durch Extrusionsdrucken, Inkjet-Drucken, syringe-printing oder Bioplotting oder Laserdrucken bearbeitet wird, wobei das Inkjet-Drucken bevorzugt Drop-on-demand Inkjet-Drucken ist.

15. Verfahren nach Anspruch 14, wobei die lebenden Zellen ausgewählt sind aus der Gruppe bestehend aus Chondrocyten, Osteoblasten, Osteoclasten, Hautepithelzellen, intestinalen Epithelzellen, Cornea-Epithelzellen, Astrocyten, Neuronen, Oligodendrocyten, glatten Muskelzellen, Endothelzellen, Cardiomyocyten, Pankreas-Inselzellen, Nieren-Epithelzellen und naiven Zellen erhalten aus der Nabelschnur, oder wobei die lebenden Zellen Stammzellen sind, ausgewählt aus der Gruppe bestehend aus embryonalen Stammzellen, somatischen Stammzellen, reprogrammierten pluripotenten somatischen Zellen, induzierten pluripotenten Zellen und amniotischen Stammzellen.

## Revendications

1. Utilisation d'une matrice comprenant un polysaccharide modifié contenant des groupes hydroxyles primaires comprenant des unités disaccharides répétitives dans lesquelles dans au moins une partie des unités disaccharides le groupe hydroxyle primaire est remplacé par des groupes fonctionnels choisis parmi des groupes carboxyles des groupes halogénures ou des groupes comprenant des atomes de soufre ou de phosphore, comme par exemple des groupes sulfates, des groupes sulfonates, des groupes phosphonates et des groupes phosphates, pour des procédés de bio-impression, **caractérisée en ce que** le polysaccharide modifié contenant des groupes hydroxyles primaires est de l'agarose.

2. Utilisation selon la revendication 1, dans laquelle le procédé de bio-impression est choisi parmi de l'impression par jet d'encre, de l'impression à la seringue ou du bioplotage et de l'impression au laser.

3. Utilisation selon la revendication 1 ou 2, dans laquelle le groupe fonctionnel est un groupe carboxyle.

4. Utilisation selon la revendication 1 ou 2, dans laquelle le groupe fonctionnel est un groupe halogénure.

5. Utilisation selon la revendication 1 ou 2, dans laquelle le groupe fonctionnel est un groupe phosphonate ou phosphate.

6. Utilisation selon la revendication 1 ou 2, dans laquelle le groupe fonctionnel est un groupe sulfate ou sulfonate.

7. Utilisation selon l'une quelconque des revendications précédentes dans laquelle la matrice comprend en outre des polysaccharides non modifiés.

8. Utilisation selon l'une quelconque des revendications précédentes dans laquelle le polysaccharide modifié ou le polysaccharide non modifié ou les deux sont choisis dans le groupe consistant en un membre de la famille des carraghénanes, de l'acide hyaluronique et de l'agarose.

9. Utilisation selon la revendication 8 dans laquelle le polysaccharide modifié et le polysaccharide non modifié sont tous deux de l'agarose.

10. Utilisation selon l'une quelconque des revendications précédentes dans laquelle dans les polysaccharides modifiés au moins 5 % des groupes hydroxyles primaires sont convertis en un groupe fonctionnel.

11. Utilisation d'une formulation de bio-encre comprenant un polysaccharide modifié contenant des groupes hydroxyle primaires, comprenant des unités disaccharides répétitives dans lesquelles dans au moins une partie des unités disaccharides le groupe hydroxyle primaire est remplacé par des groupes fonctionnels choisis parmi des groupes carboxyles, des groupes halogénures ou des groupes comprenant des atomes de soufre ou de phosphore, comme par exemple des groupes sulfates, des groupes sulfonates, des groupes phosphonates et des groupes phosphates, **caractérisée en ce que** le polysaccharide modifié contenant des groupes hydroxyle primaires est de l'agarose, et des cellules vivantes, pour de la bio-impression ou des procédés de bio-impression.

12. Utilisation selon la revendication 11, dans laquelle les cellules vivantes sont choisies dans le groupe constitué des chondrocytes, des ostéoblastes, des ostéoclastes, des cellules épithéliales cutanées, des cellules épithéliales intestinales, des cellules épithéliales cornéennes, des astrocytes, des neurones, des oligodendrocytes, des cellules musculaires lisses, des cellules endothéliales, des cardiomyocytes, des cellules des îlots pancréatiques, des cellules épithéliales rénales et des cellules naïves obtenues à partir du cordon ombilical.

13. Utilisation selon la revendication 11 ou 12, dans laquelle les cellules vivantes sont des cellules souches choisies dans le groupe constitué des cellules souches embryonnaires, des cellules souches somatiques, des cellules somatiques pluripotentes reprogrammées, des cellules pluripotentes induites et des cellules souches amniotiques.

14. Procédé de structuration tridimensionnelle de matériaux biologiques, dans lequel une formulation de bio-encre comprenant un polysaccharide modifié contenant des groupes hydroxyle primaires comprenant des unités disaccharides répétitives dans lesquelles dans au moins une partie des unités disaccharides le groupe hydroxyle primaire est remplacé par des groupes fonctionnels choisis parmi des groupes carboxyles, des groupes halogénures ou des groupes comprenant des atomes de soufre ou de phosphore, comme par exemple des groupes sulfates, des groupes sulfonates, des groupes phosphonates et des groupes phosphates, **caractérisé en ce que** le polysaccharide modifié contenant des groupes hydroxyles primaires est de l'agarose, et des cellules vivantes, est traitée par impression par extrusion, impression par jet d'encre, impression à la seringue ou biopotage ou impression laser, dans lequel l'impression par jet d'encre est de préférence une impression par jet d'encre "goutte à la demande".

15. Procédé selon la revendication 14, dans lequel les cellules vivantes sont choisies dans le groupe constitué des chondrocytes, des ostéoblastes, des ostéoclastes, des cellules épithéliales cutanées, des cellules épithéliales intestinales, des cellules épithéliales cornéennes, des astrocytes, des neurones, des oligodendrocytes, des cellules musculaires lisses, des cellules endothéliales, des cardiomyocytes, des cellules des îlots pancréatiques, des cellules épithéliales rénales et des cellules naïves obtenues à partir du cordon ombilical, ou dans lequel les cellules vivantes sont des cellules souches choisies dans le groupe constitué des cellules souches embryonnaires, des cellules souches somatiques, des cellules somatiques pluripotentes reprogrammées, des cellules pluripotentes induites et des cellules souches amniotiques.
